# EUROPEAN PATENT APPLICATION

(11) **EP 4 759 923 A1**
(43) Date of publication of application: **17.06.2026**
(21) Application number: 24852359.9
(22) Date of filing: 08.08.2024
(51) Int. Cl.: C12N 5/0775

(54) **METHOD FOR CULTURING TONSIL-DERIVED MESENCHYMAL STEM CELLS BY USING N-ACETYLCYSTEINE (NAC)**

(30) Priority: 08.08.2023 KR 20230103739
(71) Applicant: HYUNDAEMEDITECH CO., LTD., Wonju-si Gangwon-do 26347 (KR)
(72) Inventor: SEO, Young Joon, Seoul 06326 (KR)
(74) Representative: Vossius & Partner Patentanwälte Rechtsanwälte mbB
(86) International application number: PCT/KR2024/011763
(87) International publication number: WO 2025/034006

(57) **Abstract**

The present invention relates to a culture medium for tonsil-derived mesenchymal stem cells comprising N-acetylcysteine (NAC) and a method for culturing tonsil-derived mesenchymal stem cells using the same. According to the culture medium and the culturing method of the present invention, antioxidant efficacy can be enhanced by effectively reducing reactive oxygen species (ROS) during the culturing of tonsil-derived mesenchymal stem cells.

## Description

### [Technical Field]

The present invention relates to a culture medium for tonsil-derived mesenchymal stem cells comprising N-acetylcysteine (NAC) and a method for culturing tonsil-derived mesenchymal stem cells using the same. More specifically, the present invention relates to a method for culturing tonsil-derived mesenchymal stem cells capable of enhancing the antioxidant capacity of tonsil-derived mesenchymal stem cells by using a culture medium containing N-acetylcysteine.

### [Background Art]

Stem cells are cells capable of differentiating into various types of cells constituting biological tissues, and collectively refer to undifferentiated cells at a pre-differentiation stage that can be obtained from embryonic, fetal, and adult tissues. Stem cells have characteristics in that they undergo differentiation into specific cells in response to differentiation stimuli (environmental conditions), are capable of producing identical cells through cell division (self-renewal), and possess plasticity allowing differentiation into different types of cells depending on differentiation stimuli.

Stem cells may be classified, according to their differentiation potential, into totipotent stem cells, pluripotent stem cells, and multipotent stem cells.

Totipotent stem cells are cells having totipotency capable of developing into a complete individual. Cells from a fertilized egg up to the 8-cell stage possess such properties, and when these cells are isolated and implanted into the uterus, they are capable of developing into a complete individual. Pluripotent stem cells are cells capable of developing into various cells and tissues derived from the ectoderm, mesoderm, and endoderm. These cells are derived from the inner cell mass located inside a blastocyst appearing approximately 4 to 5 days after fertilization and are referred to as embryonic stem cells. Although pluripotent stem cells can differentiate into various other tissue cells, they are not capable of forming a new organism. Multipotent (or multipotential) stem cells are stem cells capable of differentiating only into cells specific to the tissues and organs in which they reside. These cells are involved in the growth and development of tissues and organs during the fetal, neonatal, and adult stages, as well as in maintaining homeostasis of adult tissues and inducing regeneration upon tissue injury. Tissue-specific multipotent cells are collectively referred to as mesenchymal stem cells.

Meanwhile, mesenchymal stem cells (MSCs) are non-hematopoietic multipotent fibroblast-like cells that exhibit the capacity to differentiate into mesodermal lineages, including osteocytes, adipocytes, and chondrocytes. MSCs can be readily isolated from a variety of tissues, including bone marrow, adipose tissue, amniotic fluid, and Wharton's jelly (substantia gelatinea funiculi umbilicalis), and possess immunomodulatory properties, thereby enabling successful allogeneic transplantation. In addition, mesenchymal stem cells can be derived from various types of tissues. However, it has been reported that mesenchymal stem cells may exhibit heterogeneity depending on differences in in vitro expansion culture conditions, differences in stem cell donors, or differences in the tissue sites from which the stem cells are isolated.

However, among mesenchymal stem cells, some types of stem cells are difficult to utilize due to significant limitations in obtaining the cells. For example, mesenchymal stem cells derived from umbilical cord blood or adipose tissue must be obtained using invasive methods. The most non-invasive method for obtaining mesenchymal stem cells is through bone marrow aspiration; however, bone marrow collection requires anesthesia and causes pain, thereby limiting its use. As an alternative, methods for obtaining cells using peripheral blood have been required to isolate patient-specific stem cells. However, peripheral blood alone yields an insufficient number of mesenchymal stem cells that can be isolated from adults, the isolation methods are not cost-effective, and even when isolation is successful, expansion to a quantity usable for cell therapy is often inefficient. Therefore, there is a need for alternative approaches with improved practicality. In addition, adult stem cells obtained from elderly patients exhibit significantly reduced proliferative capacity compared to cells obtained from younger individuals, and show decreased secretion of various factors and reduced migratory ability of stem cells toward lesions. Accordingly, there is a need to obtain cells from tissues that can be naturally isolated from younger patients or from discarded tissues. Furthermore, the cells thus obtained should be capable of being secured in quantities suitable for experimentation and should maintain differentiation potential during serial passaging.

Meanwhile, tonsil-derived mesenchymal stem cells (TMSCs) refer to undifferentiated stem cells that are derived from tonsils, which are tissues located at the inner part of the throat and the posterior part of the nasal cavity and function as lymphoepithelial immune tissues that primarily defend the body against invading substances such as bacteria. TMSCs possess self-renewal capacity and the ability to differentiate into two or more new cell types.

Tonsil-derived mesenchymal stem cells have the advantage that stem cells can be obtained without additional pain to the donor, unlike mesenchymal stem cells derived from other tissues, because the stem cells are extracted from tissues that are discarded as medical waste after tonsillectomy. In addition, compared to stem cells obtained by harvesting from bone marrow over a period of approximately two hours, tonsil-derived mesenchymal stem cells can be obtained in larger quantities and exhibit superior proliferative capacity, and thus are known to have very high stem cell productivity.

Tonsil-derived mesenchymal stem cells are superior to other mesenchymal stem cells in terms of proliferation rate, differentiation potential, and immunomodulatory capacity, and successfully maintain MSC-specific characteristics during freezing and thawing and up to a maximum of 15 passages during long-term in vitro cell culture. Accordingly, TMSCs are emerging as promising MSC candidates for clinical cell therapy.

Mesenchymal stem cells can differentiate into osteocytes, chondrocytes, and adipocytes and possess anti-inflammatory activity, immunomodulatory functions, antifibrotic activity, and the ability to regenerate damaged skin. However, while most mesenchymal stem cells are capable of differentiation only within a limited range, tonsil-derived mesenchymal stem cells have been reported to be capable of differentiating not only into mesoderm-derived cells such as muscle cells, but also into endoderm-derived cells such as parathyroid hormone-secreting cells and insulin-secreting cells, as well as ectoderm-derived cells such as motor neurons and Schwann cells. In addition, therapeutic efficacy of tonsil-derived mesenchymal stem cells for skin inflammatory diseases and chronic inflammatory bowel diseases has also been reported.

Meanwhile, reactive oxygen species (ROS) are highly reactive chemical species formed from molecular oxygen (O₂), and in biological systems, ROS are byproducts of normal oxygen metabolism. Reduction of molecular oxygen generates peroxides, and disproportionation of peroxides produces hydrogen peroxide (H₂O₂). Hydrogen peroxide may be partially reduced to form hydroxide ions and hydroxyl radicals (·OH), or may be completely reduced to water.

Reactive oxygen species are essential for cellular functions and are present at low and steady levels in normal cells. However, ROS levels can increase rapidly under environmental stress. Elevated ROS oxidize and modify certain cellular components and interfere with their original functions, thereby causing irreversible damage to DNA. This can result in severe damage to cellular structures. Such a condition is referred to as oxidative stress. Oxidative stress induces oxidative DNA damage, which subsequently upregulates p53 and triggers intrinsic mitochondrial apoptosis.

N-acetylcysteine (NAC) is a precursor of cysteine, a sulfur-containing amino acid used by cells for glutathione biosynthesis, and is known as an antioxidant molecule that exerts its effects by increasing intracellular glutathione (GSH) synthesis. The reducing effect of GSH is attributed to the direct scavenging of reactive oxygen species as well as the recycling of antioxidants that have already been consumed.

In this regard, Korean Patent Registration No. 10-1211913 discloses a culture medium composition for culturing amnion-derived mesenchymal stem cells containing N-acetyl-L-cysteine and a method for culturing amnion-derived mesenchymal stem cells using the same. Korean Patent Publication No. 10-2016-0079390 discloses a culture medium composition for culturing stem cells containing N-acetyl-L-cysteine, and PCT Patent Publication No. 2017/032811 discloses that a culture medium for embryos, gametes, or stem cells may further include acetylcysteine.

As described above, although the use of N-acetylcysteine in stem cell culture is known, there have been no reports of applying N-acetylcysteine specifically to the culturing of tonsil-derived mesenchymal stem cells. In order to obtain such mesenchymal stem cells in large quantities while maintaining stem cell performance, the culturing method is critical. The present inventors confirmed that when N-acetylcysteine is treated at a specific concentration, excellent antioxidant efficacy is exhibited in the culturing of tonsil-derived mesenchymal stem cells, thereby completing the present invention.
(Patent Document 0001) Korean Patent Registration No. 10-1211913
(Patent Document 0002) Korean Patent Publication No. 10-2016-0079390
(Patent Document 0003) PCT Patent Publication No. 2017/032811

### [Detailed Description of the Invention]

### [Technical Problem]

The present invention provides a culture medium for tonsil-derived mesenchymal stem cells comprising N-acetylcysteine, and a method for culturing tonsil-derived mesenchymal stem cells using the same.

### [Technical Solution]

The present invention provides a culture medium for tonsil-derived mesenchymal stem cells comprising N-acetylcysteine (NAC) as an active ingredient.

In addition, the present invention provides a method for culturing tonsil-derived mesenchymal stem cells, comprising a step of culturing tonsil-derived mesenchymal stem cells in a stem cell culture composition comprising N-acetylcysteine (NAC) as an active ingredient.

### [Advantageous Effects of the Invention]

According to the culture medium and the culturing method of the present invention, reactive oxygen species (ROS) can be effectively reduced during the culturing of tonsil-derived mesenchymal stem cells, thereby enhancing antioxidant efficacy.

### [Brief Description of the Drawings]

Fig. 1 shows the results of measuring cell viability after treating tonsil-derived human mesenchymal stem cells with NAC at concentrations of 2, 4, 6, 8, 10, 15, and 100 mM for 24 hours.
Fig. 2 shows the results of measuring absorbance in one embodiment of the present invention (Experimental Example 1).
Fig. 3 shows the results of measuring cell viability after pretreating tonsil-derived human mesenchymal stem cells with NAC in one embodiment of the present invention (Experimental Example 1).
Fig. 4 shows the results of measuring absorbance in one embodiment of the present invention (Experimental Example 2).
Fig. 5 shows the results of measuring reactive oxygen species (ROS) after pretreating tonsil-derived human mesenchymal stem cells with NAC in one embodiment of the present invention (Experimental Example 2).

### [Best Mode for Carrying Out the Invention]

Hereinafter, embodiments of the present invention will be described in detail so that a person having ordinary skill in the art to which the present invention pertains can easily carry out the invention. The embodiments of the present invention are provided to more completely explain the present invention to those having average knowledge in the art. Accordingly, the embodiments of the present invention may be modified into various other forms, and the scope of the present invention is not limited to the embodiments described below.

Throughout the specification of the present invention, when a part is described as "comprising" a certain component, this means that, unless otherwise specifically stated, it does not exclude other components and may further include other components.

One embodiment of the present invention provides a culture medium for tonsil-derived mesenchymal stem cells comprising N-acetylcysteine (NAC) as an active ingredient.

In the present invention, N-acetylcysteine, also known as 2-acetamido-3-sulfanylpropanoic acid or NAC, has the following chemical structural formula:

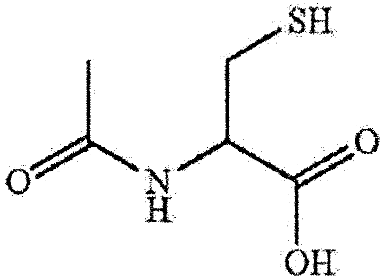

In the present invention, the term "tonsil-derived stem cells" refers to undifferentiated stem cells derived from tonsils, which are tissues located at the inner part of the throat and the posterior part of the nasal cavity and function as lymphoepithelial immune tissues that primarily defend the body against invading substances such as bacteria, and which possess self-renewal capability and the ability to differentiate into two or more new cell types.

Tonsil-derived mesenchymal stem cells can be isolated from tonsils according to methods known in the art. For example, they may be isolated from tonsils according to the method described in Korean Patent No. 10-1508413. The culture medium for tonsil-derived mesenchymal stem cells may be a culture medium, a culture supernatant, or a concentrate thereof obtained after culturing tonsil-derived mesenchymal stem cells and removing the cells, or a lyophilized product thereof.

In the present invention, the term "culture medium" refers to a composition containing nutrients necessary to maintain the growth and viability of cells in vitro, and tonsil-derived mesenchymal stem cells may be conventionally cultured using a stem cell culture medium.

The culture medium for tonsil-derived mesenchymal stem cells may be obtained by subculturing tonsil-derived mesenchymal stem cells isolated from tonsil tissue in a serum-containing medium, followed by subculturing in a serum-free medium. The resulting medium may be used as it is, or the supernatant obtained after removing stem cells and macromolecules by centrifugation or filtration may be used. In addition, the obtained supernatant may be used as it is or may be concentrated and used in the form of a concentrate.

Culture media and culture conditions for culturing tonsil-derived mesenchymal stem cells are well known in the art to which the present invention pertains, and may be appropriately selected or modified and used by a person having ordinary skill in the art.

The serum-containing medium may be a medium suitable for maintaining and preserving the same cell type as tonsil-derived mesenchymal stem cells, and may be, for example, a DMEM medium supplemented with serum. The serum may be fetal bovine serum (FBS), but is not limited thereto, and may be present in an amount of 1 to 10 wt% based on the total weight of the serum-containing medium. If necessary, the medium may further comprise antibiotics, antifungal agents, and mycoplasma inhibitors, and these may be included in an amount of about 1 wt% based on the total weight of the medium. The antibiotics may include those commonly used in cell culture, such as penicillin-streptomycin; the antifungal agents may include amphotericin B (fungizone); and the mycoplasma inhibitors may include gentamicin, ciprofloxacin, tylosin, and the like, without being limited thereto.

The culturing in the serum-free medium may be performed by removing the serum-containing medium and washing the cells with a phosphate-buffered solution, followed by culturing in the serum-free medium.

The NAC may be contained at a concentration of 1 to 100 mM. More specifically, the concentration may be 1 to 90 mM, 1 to 80 mM, 1 to 70 mM, 1 to 60 mM, 1 to 50 mM, 1 to 40 mM, or 1 to 30 mM; still more specifically, 5 to 30 mM, 5 to 25 mM, or 5 to 20 mM; and even more specifically, 5 to 19 mM, 5 to 18 mM, 5 to 17 mM, 5 to 16 mM, 5 to 15 mM, 6 to 15 mM, 7 to 15 mM, 8 to 15 mM, 9 to 15 mM, 9 to 14 mM, 9 to 13 mM, 9 to 12 mM, 9 to 11 mM, or 10 mM, but is not limited thereto.

The stem cell culture medium is characterized by having enhanced antioxidant efficacy, and in particular, exhibits antioxidant efficacy by reducing reactive oxygen species (ROS), but is not limited thereto.

In addition, another embodiment of the present invention provides a method for culturing tonsil-derived mesenchymal stem cells, comprising a step of culturing tonsil-derived mesenchymal stem cells in a stem cell culture composition comprising N-acetylcysteine (NAC) as an active ingredient.

The culturing method is characterized in that antioxidant efficacy is enhanced, and in particular, exhibits antioxidant efficacy by reducing reactive oxygen species (ROS), but is not limited thereto.

### [Mode for Carrying Out the Invention]

Hereinafter, the present invention will be described in more detail with reference to Examples and Experimental Examples. However, the following Examples and Experimental Examples are provided for the purpose of facilitating understanding of the present invention, and are not intended to limit the scope of the present invention.

### <Example 1> Preparation of NAC

For pretreatment of tonsil-derived human MSCs, 163.19 mg of N-acetyl-L-cysteine powder (molecular weight: 163.19 g/mol) was added to 10 mL of PBS and mixed, and the pH was adjusted to 7.4 by adding NaOH.

### <Experimental Example 1> Evaluation of Cell Viability

### 1-1. Determination of NAC Pretreatment Concentration

In order to determine the concentration of NAC for pretreatment of tonsil-derived human MSCs, the tonsil-derived human MSCs were pretreated with NAC prepared in Example 1 at concentrations of 2, 4, 6, 8, 10, 15, and 100 mM for 24 hours, and then cell viability was evaluated.

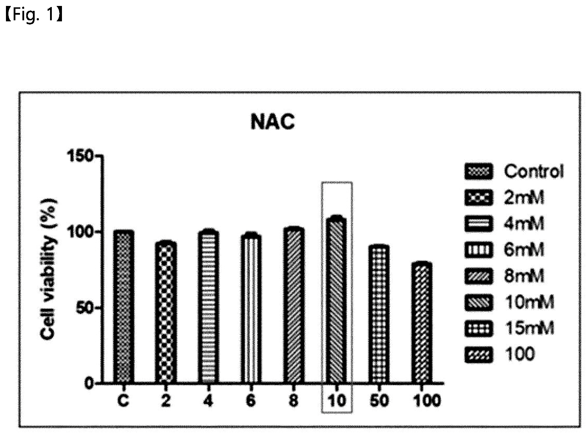

As a result, as shown in Fig. 1, no cytotoxicity was observed at a concentration of 10 mM after pretreatment with NAC for 24 hours.

Accordingly, the concentration of NAC to be used in subsequent experiments was determined to be 10 mM.

### 1-2. Evaluation of Cell Viability upon NAC Treatment in Tonsil-Derived Human MSCs

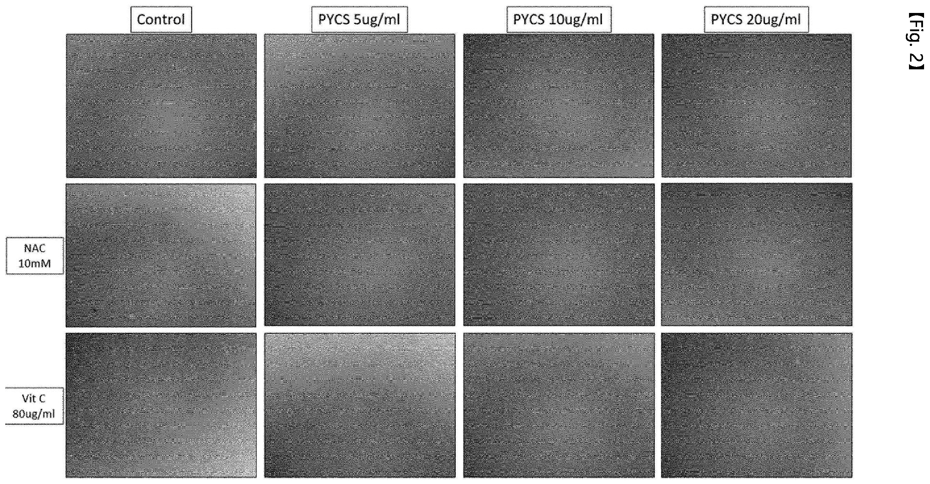

In order to evaluate cell viability upon NAC treatment, tonsil-derived human MSCs (hMSC-T, passage 6) were seeded in a culture medium at a density of 1×10³ cells/well. On the following day, NAC was treated at concentrations of 10 mM and 20 mM for 24 hours. Thereafter, the cells were washed with PBS and the medium was replaced, followed by treatment with PYBR at concentrations of 5, 10, and 20 µg/mL After 24 hours, the cells were washed three times with PBS and transferred to fresh medium (100 µL) containing 10 µL of WST solution, and incubated at 37°C for 2 hours in the dark. Absorbance at 450 nm was then measured to evaluate cell viability (Fig. 2). A group not treated with NAC was used as a positive control.

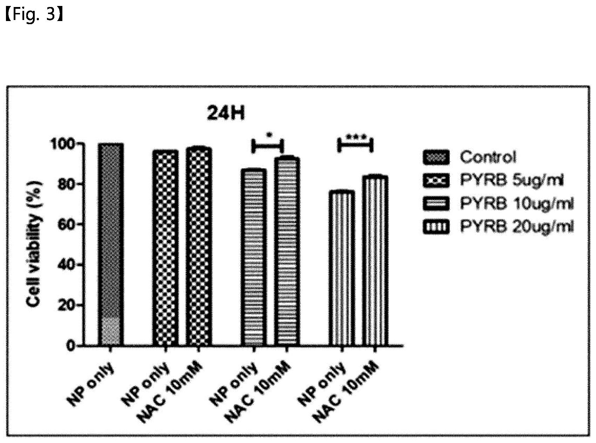

As a result, as shown in Fig. 3, treatment with PYBR alone resulted in a decrease in cell viability by about 20-30%, whereas pretreatment with NAC significantly restored cell viability.

Accordingly, it was confirmed that treatment of tonsil-derived human MSCs with NAC can improve cell viability.

### <Experimental Example 2> Evaluation of Antioxidant Activity (ROS Assay)

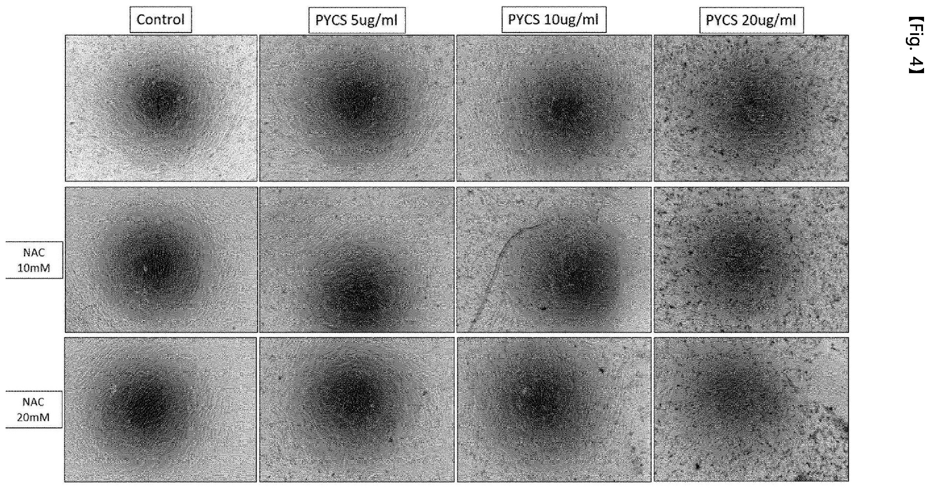

In order to evaluate antioxidant activity upon NAC treatment, tonsil-derived human MSCs (hMSC-T, passage 6) were seeded in a culture medium at a density of 1×10⁴ cells/well. On the following day, NAC was treated at concentrations of 10 mM and 20 mM for 24 hours. Thereafter, the cells were washed with PBS and the medium was replaced, followed by treatment with PYBR at concentrations of 5, 10, and 20 µg/mL After 24 hours, the cells were washed three times with PBS and incubated in 100 µL of ROS working solution at 37°C for 30 minutes in the dark. Absorbance was then measured at 488/525 nm (Fig. 4). A group not treated with NAC was used as a positive control.

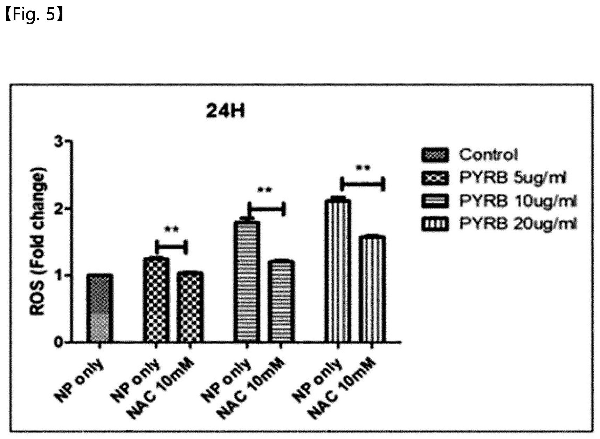

As a result, as shown in Fig. 5, treatment with PYBR alone resulted in an increase in ROS levels, whereas pretreatment with NAC significantly reduced ROS levels.

Accordingly, it was confirmed that treatment of tonsil-derived human MSCs with NAC reduces ROS and thus provides excellent antioxidant effects.

### [Industrial Applicability]

According to the culture medium and the culturing method of the present invention, antioxidant efficacy can be enhanced by effectively reducing reactive oxygen species (ROS) during the culturing of tonsil-derived mesenchymal stem cells.

## Claims

1. A culture medium for tonsil-derived mesenchymal stem cells, comprising N-acetylcysteine (NAC) as an active ingredient.

2. The culture medium of claim 1, wherein the NAC is contained at a concentration of 1 to 100 mM.

3. The culture medium of claim 1, wherein the culture medium has enhanced antioxidant efficacy.

4. A method for culturing tonsil-derived mesenchymal stem cells, comprising a step of culturing tonsil-derived mesenchymal stem cells in a stem cell culture composition comprising N-acetylcysteine (NAC) as an active ingredient.

5. The method of claim 4, wherein antioxidant efficacy is enhanced by the culturing method.

6. The method of claim 4, wherein the N-acetylcysteine (NAC) is contained at a concentration of 1 to 100 mM.
